Europäisches Patentamt

(19) **European Patent Office**

Office européen des brevets

(11) Numéro de publication: **0 283 635**
**B1**

(12) **FASCICULE DE BREVET EUROPEEN**

(45) Date de publication du fascicule du brevet:
**19.12.90**

(51) Int. Cl.⁵: **B01J 23/78**, C07C 29/15

(21) Numéro de dépôt: **87870036.8**

(22) Date de dépôt: **19.03.87**

(54) Catalyseurs polymétalliques, leur préparation et leur utilisation.

(43) Date de publication de la demande:
**28.09.88 Bulletin 88/39**

(45) Mention de la délivrance du brevet:
**19.12.90 Bulletin 90/51**

(84) Etats contractants désignés:
**AT BE CH DE ES FR GB IT LI LU NL SE**

(56) Documents cités:
**EP-A- 0 005 492**
**EP-A- 0 099 715**
**EP-A- 0 100 607**
**FR-A- 943 408**
**FR-A- 1 011 673**
**FR-A- 1 074 045**
**US-A- 2 539 847**

(73) Titulaire: **De Belgische Staat L'Etat Belge représenté par le Secrétaire général des Services de Programmation, de la politique scientifique Rue de la Science, 8, B-1040 Bruxelles(BE)**

(72) Inventeur: **Noels, Alfred, Avenue de la Paix, 50/063, B-4030 Liege-Grivegnee(BE)**
Inventeur: **Hubert, André, Rue N. Fossoul, 67, B-4208 Boncelles(BE)**
Inventeur: **Demonceau, Albert, Neuve Cour, 2, B-4661 Clermont-Thimister(BE)**
Inventeur: **Teyssié, Philippe, Avenue Bois Impérial de Rognac, 85, B-4121 Neuville-en-Condroz(BE)**

(74) Mandataire: **Gaspar, Florent et al, Bureau VANDER HAEGHEN 63, avenue de la Toison d'Or, B-1060 Bruxelles(BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 283 635 B1

**Description**

La présente invention est relative à des catalyseurs polymétalliques, à leur préparation et à leur utilisation pour la fabrication d'alcools, en particulier de mélanges d'alcools aliphatiques linéaires en $C_1$ à $C_8$ à partir de gaz de synthèse.

Il est connu par le document FR-A-2.523.957 de fabriquer des alcools primaires par réaction d'oxydes de carbone (principalement du monoxyde de carbone) et d'hydrogène en présence de catalyseurs renfermant au moins quatre métaux essentiels, à savoir le cuivre, le cobalt, l'aluminium, ainsi qu'au moins un métal (A) alcalin ou alcalino-terreux, les rapports atomiques entre ces métaux étant les suivants :
Cu/Co = 0,1 à 5; Al/Co = 0,7 à 4; métaux A/Co = 0,05 à 1,5

Ces catalyseurs connus peuvent éventuellement contenir, en outre, du zinc, ainsi qu'un métal M choisi parmi le manganèse, le fer, le vanadium et le rhénium et/ou un métal N choisi parmi le scandium, l'yttrium, le thorium, le zirconium et les métaux des terres rares, de même qu'éventuellement du chrome.

On connaît par ailleurs, par le document EP-A-0 100 607, des compositions catalytiques convenant pour la production d'alcools à partir de gaz de synthèse, ces compositions contenant essentiellement :

(a) du cobalt,
(b) au moins un métal choisi parmi le cuivre, l'argent, le gallium, le zirconium, le zinc et le thorium,
(c) au moins un métal choisi parmi le palladium, le platine et le nickel, et
(d) au moins un métal alcalin, le rapport atomique des éléments (a) : (b) : (c) étant de 100 : 1 à 400 : 1 à 500, le ou les métaux alcalins pouvant former jusqu'à 5 % en poids de la composition.

Les exemples donnés dans ce document EP-A-0 100 607 concernent des compositions catalytiques contenant du cuivre, du cobalt, du palladium et du potassium. La présence de platine, de palladium ou de nickel dans des compositions catalytiques polymétalliques pour la production d'alcools au départ de gaz de synthèse n'est pas favorable. Ainsi, la présence de platine qui, à l'instar du palladium, est un métal coûteux, favorise la production d'hydrocarbures au détriment des alcools. Quant au nickel et au palladium, il diminue la sélectivité en alcools supérieurs à deux atomes de carbone et plus, en favorisant la production de méthanol. En outre, il est connu que le nickel s'échappe du système réactionnel sous forme de nickel carbonyle qui est un composé extrêmement toxique, ce qui entraîne des variations des performances catalytiques et des dangers de pollution.

On connaît encore, par le document FR-A-1.011.673, des compositions catalytiques pour l'hydrogénation d'oxyde de carbone en hydrocarbures, ces compositions contenant du cobalt, de l'oxyde de thorium et éventuellement de l'oxyde de magnésium sur un support formé de Kieselguhr.

On connaît enfin, par le document FR-A-1.074.045, un procédé d'hydrogénation catalytique d'oxyde de carbone en vue d'obtenir soit des produits de synthèse à forte proportion de composés oxygénés, de préférence d'alcools aliphatiques, soit des produits constitués en majeure partie d'hydrocarbures supérieurs ou inférieurs, ce procédé utilisant des catalyseurs constitués par du cuivre, et des métaux du groupe du fer, tels que le cobalt et le fer, éventuellement en présence d'activants connus, par exemple des bases alcalino-terreuses, des alcalis et des oxydes de zinc, de chrome, d'aluminium et de thorium. Les compositions catalytiques utilisées dans le procédé selon le document FR-A-1.074.045 contiennent toutes du cuivre et du cobalt ou du fer, abstraction faite des activants. Ces compositions permettent l'obtention d'alcools à partir de gaz de synthèse, sans que leur sélectivité en alcools supérieurs au méthanol soit montrée.

La présente invention a pour objet une classe de catalyseurs polymétalliques exempts de platine, de palladium, de nickel, de cuivre, de fer et d'aluminium, les catalyseurs de cette classe ayant une bonne stabilité et des durées de vie particulièrement intéressantes, tout en présentant une sélectivité remarquable en alcools supérieurs au méthanol, cette sélectivité étant notamment nettement plus élevée que celle des catalyseurs selon le document FR-A-2.523.957.

Ainsi, les alcools primaires saturés obtenus à l'aide des catalyseurs suivant l'invention contiennent une proportion considérable d'alcools en $C_2$ à $C_6$ (schématiquement indiqués par $C_2{}^+OH$ dans la suite du présent mémoire).

Les mélanges d'alcools obtenus en utilisant les catalyseurs présentent de nombreuses utilisations. Ainsi, pour former des carburants mixtes hydrocarbures-alcools destinés à des moteurs à combustion interne, il est particulièrement intéressant de disposer de mélanges d'alcools contenant une proportion importante d'alcools aliphatiques primaires en $C_2$ à $C_6$. En effet, la diminution ou la suppression du plomb dans l'essence destinée aux moteurs des véhicules automobiles (environ 55 % du volume total d'essence pour véhicules à moteurs devraient être exempts de plomb vers 1990) implique une augmentation de plusieurs points de l'indice d'octane des carburants. Le méthanol et les alcools supérieurs ($C_2{}^+OH$), principalement l'éthanol, possèdent des indices d'octane élevés, mais ces alcools supérieurs ont une meilleure compatibilité avec les hydrocarbures que le méthanol et facilitent également l'incorporation de ce même méthanol aux hydrocarbures, ce qui prouve l'intérêt de l'obtention de mélanges d'alcools contenant des proportions importantes d'alcools supérieurs $C_2{}^+OH$ par rapport au méthanol.

Les catalyseurs suivant la présente invention renferment essentiellement du thorium, du cobalt et au moins un métal (A) alcalin éventuellement associé à au moins un métal alcalino-terreux choisis dans le

2

groupe formé par le lithium (Li), le sodium (Na), le potassium (K), le rubidium (Rb), le béryllium (Be), le magnésium (Mg), le calcium (Ca), le strontium (Sr) et le baryum (Ba).

Les métaux alcalins et/ou alcalino-terreux préférés sont le sodium, le potassium, le magnésium, le calcium et/ou le baryum.

Selon une particularité de l'invention, les catalyseurs contiennent également du zinc dans des proportions relatives très variables par rapport aux métaux de base, la présence de cet élément favorisant la bonne tenue du catalyseur.

Les catalyseurs suivant l'invention contenant ou non du zinc peuvent être déposés, sous forme de mélanges uniformes des métaux précités, sur divers supports minéraux, par exemple des oxydes métalliques ou de non métaux (silice, alumine, $TiO_2$, $ZrO_2$, des silicates, alumino-silicates, calciosilicates, etc.).

Conformément à la présente invention, les rapports atomiques entre les métaux présents dans les catalyseurs sont les suivants :

$Th/Co$ = 0,2 à 10, de préférence 0,4 à 2

$Th/A$ = 0,01 à 10, de préférence 0,05 à 1

$Th/Zn$ = 0,001 à 1, de préférence 0,01 à 0,5

Des exemples de compositions de catalyseurs polymétalliques suivant l'invention sont donnés dans le tableau I suivant, où du sodium est utilisé comme métal alcalin (moles %).

TABLEAU I

| Th | Co | Na | Zn |
|---|---|---|---|
| 70 | 5 | 25 | 0 |
| 20 | 30 | 50 | 0 |
| 10 | 60 | 30 | 0 |
| et de préférence | | | |
| 40 | 15 | 45 | 0 |
| 65 | 5 | 25 | 5 |
| 20 | 25 | 50 | 5 |
| 10 | 55 | 30 | 5 |
| 33 | 3 | 13 | 50 |
| 6 | 29. | 15 | 50 |
| 6,5 | 1 | 2,5 | 90 |
| 2 | 2,7 | 5,3 | 90 |
| 1 | 6 | 3 | 90 |
| et de préférence | | | |
| 12 | 5 | 13 | 70 |

Dans le tableau I, le sodium peut être remplacé par au moins un autre métal alcalin éventuellement associé à au moins un métal alcalino-terreux choisis parmi ceux indiqués plus haut dans des proportions analogues à celles du sodium.

L'addition éventuelle d'un métal alcalino-terreux à la combinaison catalytique de base permet avantageusement d'ajuster le rapport des alcools $C_2^+OH$ au méthanol, (donc de contrôler dans certaines limites la croissance des chaînes des produits oxygénés), la présence des métaux alcalino-terreux favorisant généralement la formation du méthanol (comme on le montrera plus loin - exemples 15 et 17 ci-après).

La présence de thorium dans les catalyseurs polymétalliques suivant la présente invention a pour effet principal une bonne reproductibilité des performances catalytiques. En l'absence de thorium, les catalyseurs suivant l'invention montrent fréquemment un comportement aléatoire peu reproductible, surtout en ce qui concerne le rapport hydrocarbures/alcools.

La production d'hydrocarbures au détriment de produits oxygénés a tendance à augmenter en l'absence de thorium.

Ces phénomènes défavorables constatés avec des catalyseurs polymétalliques exempts de thorium sont illustrés plus loin (voir exemple d'utilisation 26).

La présente invention est également relative à un procédé de préparation des catalyseurs suivant l'invention.

Ce procédé vise à obtenir des catalyseurs homogènes au départ de solutions (par définition, homogènes) des divers métaux composant les catalyseurs, ces solutions étant transformées en une matière solide, soit par évaporation, soit par dépôt sur un support solide, la phase solide obtenue étant avantageusement finement broyée et activée par traitement thermique.

Conformément à la présente invention, on prépare les nouveaux catalyseurs par un procédé dans lequel on dissout ensemble ou séparément dans au moins un solvant commun ou dans des solvants miscibles l'un à l'autre des composés contenant respectivement du thorium, du cobalt, au moins un métal alcalin ou alcalino-terreux et éventuellement du zinc, en utilisant des proportions telles des divers composés que la solution finale contienne les proportions relatives voulues desdits métaux, on mélange intimement la ou les solutions obtenues, on provoque la précipitation ou le dépôt de dérivés de ces métaux éventuellement sur un support solide insoluble préalablement mélangé à la solution finale, et on active éventuellement la mélange catalytique solide obtenu par traitement thermique.

Dans une première forme de réalisation du procédé d'obtention des catalyseurs suivant l'invention, on opère en milieu acide selon les étapes suivantes :
- on dissout un sel soluble de thorium dans de l'eau éventuellement additionnée d'un acide carboxylique organique, de manière à obtenir une solution A ;
- on dissout dans un mélange d'eau et d'un acide carboxylique organique, dans lequel au moins un métal alcalin a été préalablement dissous, un sel soluble de cobalt, de manière à obtenir une solution B ;
- on dissout éventuellement du zinc métallique ou un composé de zinc dans de l'eau et/ou un acide carboxylique organique, de manière à obtenir une solution C ;
- on mélange les solutions A, B et éventuellement C en les agitant de préférence pendant plusieurs heures, après y avoir éventuellement ajouté un support solide insoluble ;
- on évapore la phase liquide sous vide ;
- éventuellement on broie finement le mélange solide obtenu, et
- éventuellement on active la matière solide par chauffage sous vide ou dans un courant de gaz pendant plusieurs heures à une température comprise entre 200 et 1000°C.

Comme sel soluble de thorium, on peut utiliser avantageusement du formiate de thorium ou du nitrate de thorium hydraté, tandis que comme sel de cobalt, on utilise de préférence du carbonate, du nitrate ou de l'acétate de cobalt hydraté.

Comme composés de métal alcalin et alcalino-terreux, on utilise avantageusement un carbonate.

Quant au zinc, il est, de préférence, utilisé sous forme de zinc métallique en poudre ou d'oxyde de zinc.

Comme acide servant de solvant éventuellement en mélange avec de l'eau, on utilise, de préférence, de l'acide formique ou de l'acide acétique.

Dans une seconde forme de réalisation du procédé de préparation des catalyseurs suivant la présente invention, on opère comme suit :
- on dissout un composé de thorium et un composé de cobalt dans un hydrocarbure éventuellement additionné d'un peu d'eau;
- on chauffe la solution obtenue et, à cette solution agitée, on ajoute soit un support solide et au moins un sel de métal alcalin éventuellement associé à au moins un métal alcalino-terreux, soit un support solide préalablement alcalinisé ;
- on poursuit le chauffage et l'agitation du mélange ;
- on évapore la phase liquide ;
- on soumet éventuellement la matière solide à un fin broyage, et
- on l'active éventuellement par chauffage sous vide ou dans un courant de gaz pendant plusieurs heures à une température comprise entre 200 et 1000°C.

Comme hydrocarbure, on peut utiliser du benzène ou de l'heptane, dans lequel on dissout un composé soluble de thorium choisi, de préférence, parmi l'acétylacétonate de thorium et le formiate de sodium, ainsi qu'un composé soluble de cobalt constitué de préférence par du cobalt carbonyle.

La solution contenant du thorium et du cobalt est chauffée à une température de 25 à 275°C, de préférence à environ 70°C.

L'évaporation de la phase organique liquide s'effectue, de préférence, sous vide, dans un évaporateur rotatif ou par lyophilisation.

Dans une troisième forme de réalisation du procédé de préparation de catalyseurs suivant l'invention, on dissout successivement dans de l'eau un sel de thorium, un sel de cobalt, au moins un sel d'un métal alcalin éventuellement associé à au moins un métal alcalino-terreux et éventuellement un sel de zinc. Après addition éventuelle d'un support solide insoluble, on agite la solution pendant plusieurs heures et on élimine la phase aqueuse, de préférence par lyophilisation.

L'invention est encore relative à un procédé de synthèse d'un mélange d'alcools en $C_1$ à $C_8$. Ce procédé est essentiellement caractérisé en ce qu'on fait passer sous pression un gaz de synthèse contenant de l'oxyde de carbone et de l'hydrogène dans des proportions relatives $CO/H_2$ comprises entre 0,2 et 5, de préférence entre 0,5 et 2, sur un catalyseur suivant l'invention, à une température comprise entre 250 et 420°C, de préférence entre 300 et 350°C.

La pression totale du mélange de CO et $H_2$ peut varier entre 3 MPa et 500 MPa, de préférence entre 4 et 10 MPa à la température ambiante.

Le procédé de synthèse de mélanges d'alcools à partir de gaz de synthèse peut s'effectuer de manière discontinue dans un autoclave ou de manière continue dans un système à circulation de gaz sous pression, avec trappage ou non des produits de réaction et recyclage du gaz de synthèse non utilisé après réajustement de ce gaz de façon qu'il présente le rapport $CO/H_2$ désiré.

D'autres particularités de l'invention ressortiront des exemples illustratifs et non limitatifs suivants.

Les exemples 1 à 13 suivants décrivent la préparation de catalyseurs polymétalliques suivant la présente invention.

EXEMPLE DE PREPARATION 1 : Catalyseur Th-Co-Na

Pour obtenir un catalyseur ternaire présentant des rapports Th/Co de 0,67 et Th/Na de 0,25, on a procédé comme suit :
On a d'abord préparé deux solutions distinctes A et B.

Solution A :

0,76 g de carbonate de sodium ont été mis en réaction dans 20 ml d'acide formique à 98 %. 0,5 g de carbonate de cobalt hydraté ont ensuite été mis en suspension dans cette solution. On a ajouté alors 20 ml d'eau désionisée sous bonne agitation, ce qui a amené la dissolution du sel de cobalt. L'agitation a été poursuivie pendant 1 heure.

Solution B :

1,04 g de formiate de thorium ont été mis en solutions dans 20 ml d'eau désionisée.
Les solutions A et B ont alors été mélangées et l'agitation a été poursuivie pendant au moins 1 heure.
Le mélange résultant a été activé thermiquement par chauffage sous vide à 220°C pendant 1 heure puis à 330°C sous vide pendant 1 heure.

EXEMPLE DE PREPARATION 2 : Catalyseur Th-Co-Na-Zn

Pour obtenir un catalyseur quaternaire présentant des rapports Th/Co de 0,67 et Th/Na de 0,25, on a procédé comme suit. On a d'abord préparé deux solutions distinctes A et B.

Solution A :

0,5 g de carbonate de sodium ont été mis en réaction dans 20 ml d'acide formique à 98 %. 0,5 g de carbonate de cobalt hydraté ont ensuite été mis en suspension dans cette solution. On a ajouté alors 20 ml d'eau désionisée sous bonne agitation, ce qui a amené la dissolution du sel de cobalt. L'agitation a été poursuivie pendant 1 heure.

Solution B :

1,04 g de formiate de thorium ont été mis en solution dans 20 ml d'eau désionisée.
Les solutions A et B ont alors été mélangées et l'agitation a été poursuivie pendant au moins 1 heure.
4,0 g d'oxyde de zinc ont ensuite été introduits dans la solution homogène Co-Th-Na et l'agitation poursuivie pendant une nuit.
Le mélange résultant a été activé thermiquement par chauffage sous vide à 220°C pendant 1 heure, puis à 330°C pendant 1 heure.

EXEMPLE DE PREPARATION 3 : Catalyseur Th-Co-Li-Zn

En vue d'obtenir un catalyseur avec des rapports atomiques Th/Co de 1 de Th/Li de 0,47, on a procédé comme suit :

Solution C :

3,26 g de zinc en poudre ont été dissous dans un mélange de 35 ml d'acide formique à 98 % et de 70 ml d'eau désionisée.

La température du mélange a été maintenue à 60-70°C jusqu'à dissolution totale. On a ajouté alors 100 ml d'eau. Dès que la solution C ainsi préparée était revenue à la température ambiante, on y a ajouté les solutions A et B préparées comme décrit dans l'exemple 1, les proportions relatives en métaux étant
- pour la solution A : 0,19 g de carbonate de lithium et 0,33 g de carbonate de cobalt
- pour la solution B : quantités identiques à celles de l'exemple 1.
Pour le reste, on a opéré comme dans l'exemple 1.

EXEMPLE DE PREPARATION 4 : Catalyseur Th-Co-Ca-Na

Pour obtenir un catalyseur présentant des rapports atomiques Th/Co de 8, Th/Ca de 1 et Th/Na de 1, on a procédé comme suit :
1,87 g de nitrate de thorium hydraté ont été mis en solution dans 150 ml d'une solution d'acide acétique à 50 % pour obtenir une solution A. Une seconde solution B acétique (50 %) dans laquelle on avait préalablement introduit 0,36 g de carbonate de sodium, 0,340 g de carbonate de calcium et 0,093 g d'acétate de cobalt hydraté a été ajoutée à la solution initiale contenant du thorium portée à 60°C.
Le mélange obtenu a été agité à 60-70°C pendant 1 heure, puis le liquide à été évaporé sous vide.

EXEMPLE DE PREPARATION 5 : Catalyseur Th-Co-K-Zn sur support

Pour obtenir un catalyseur ayant des rapports atomiques Th/Co = 0,2, Th/K = 0,05 et Th/Zn = 0,5, on a procédé comme suit :
0,312 g de nitrate de thorium hydraté ont été mis en solution dans 50 ml d'acide formique à 50 % pour obtenir une solution A. Cette solution A a été ajoutée à 50 ml d'une solution B d'acide formique à 50 % contenant 0,730 g de nitrate de cobalt hydraté, 0,70 g de carbonate de potassium et 0,09 g d'oxyde de zinc.
Le mélange des solutions A et B a été porté à 90°C pendant 1 heure. Après refroidissement, 4 g d'argile bentonite K-10 (Süd Chemie, RFA) ont été ajoutés à la solution. Après vigoureuse agitation pendant une nuit, le liquide a été évaporé sous vide.

EXEMPLE DE PREPARATION 6 : Catalyseur Th-Co-K-Mg (Th/Co=1; Th/K=0,25; Th/Mg=0,10)

On a opéré selon le même mode opératoire et avec les proportions relatives des métaux dans l'acide formique à 50 % suivantes :
nitrate de cobalt hydraté : 0,50 g,
nitrate de thorium hydraté : 1,0 g,
carbonate de potassium : 0,995 g et
oxyde de magnésium : 1,45 g.

EXEMPLE DE PREPARATION 7 : Catalyseur Th-Co-Na-Li-Zn sur support

Pour obtenir ce catalyseur avec des rapports atomiques Th/Co de 0,5, Th/Na + Li de 0,05 et Th/Zn de 0,5 on a procédé comme suit :
Trois solutions ont été préparées de la manière suivante :

Solution A :

1,59 g de carbonate de sodium et 0,74 g de carbonate de lithium ont été mis en réaction dans 20 ml d'acide formique à 98 %. 0,68 g de carbonate de cobalt hydraté ont ensuite été mis en suspension dans cette solution. On a ajouté alors 20 ml d'eau désionisée sous bonne agitation, ce qui a provoqué la dissolution du sel de cobalt.

Solution B :

1,07 g de formiate de thorium ont été mis en solution dans 20 ml d'eau désionisée.

Solution C :

0,408 g d'oxyde de zinc ont été mis en solution dans 100 ml d'une solution d'acide formique à 40 %.

Les solutions A, B et C ont alors été mélangées sous agitation vigoureuse et 4,0 g d'alumine à grande surface spécifique (par exemple, Ventron alpha, 40 microns, Ventron, U.S.A.). Après une nuit d'agitation, le liquide a été évaporé sous vide.

EXEMPLE DE PREPARATION 8 : Catalyseur Th-Co-Na-Zn

Pour obtenir un catalyseur ayant des rapports atomiques Th/Co = 1,0 et Th/Na = 0,2, on a procédé comme suit :

A 20 ml de benzène sec, on a ajouté 0,17 g de cobalt carbonyle [$Co_2(CO)_8$] et 0,63 g d'acétylacétonate de thorium. La solution obtenue a été chauffée à 70°C et agitée vigoureusement pendant 24 heures. On a ensuite ajouté 2 g d'oxyde de zinc de 0,26 g de carbonate de sodium finement broyés. Le chauffage et l'agitation ont ensuite encore été maintenus pendant 24 heures. Après lyophilisation du solvant, la poudre résiduelle a été utilisée telle quelle.

EXEMPLE DE PREPARATION 9 : Catalyseur Co-Na-Zn

On a opéré comme dans l'exemple 8, si ce n'est qu'on n'a pas utilisé d'acétylacétonate de thorium.

EXEMPLE DE PREPARATION 10 : Catalyseur Th-Co-Na-Zn

Pour obtenir un catalyseur ayant des rapports atomiques Th/Co de 1,0 et Th/Na de 0,2, on a procédé comme dans l'exemple 8, excepté qu'on a utilisé 20 ml d'heptane sec au lieu de benzène et que le système catalytique a été calciné à l'air à 600°C pendant 5 heures, avant son utilisation.

EXEMPLE DE PREPARATION 11 : Catalyseur Th-Co-Na-Zn

Pour obtenir un catalyseur ayant un rapport atomique Th/Co de 0,5 et un rapport atomique Th/Na de 0,025, on a opéré comme dans l'exemple 8, si ce n'est qu'on a ajouté 0,315 g d'acétylacétonate de thorium et 1,06 g de $Na_2CO_3$.

EXEMPLE DE PREPARATION 12 : Catalyseur Th-Co-Na-Zn Rapports Th/Co = 0,5 et Th/Na = 0,2.

On a procédé comme dans l'exemple 8, si ce n'est qu'on a utilisé 0,17 g de cobalt carbonyle, 0,375 g d'acétylacétonate de thorium et 5 g de ZnO.

EXEMPLE DE PREPARATION 13 : Catalyseur Th-Co-Na-Zn Rapports Th/Co/Zn = 1/2/2 et Th/Na = 0,2.

A 20 ml d'eau, on a ajouté successivement 0,22 g d'acétate de zinc, 0,12 g de carbonate de cobalt, 0,315 g d'acétylacétonate de thorium, 0,26 g de carbonate de sodium et 1 g de bentonite (Tixoton).

La solution a été agitée pendant 24 heures à la température ordinaire et l'eau a été ensuite lyophilisée. La poudre résultante a été séchée par formation d'un mélange azéotropique avec du benzène et ensuite utilisée sans autre traitement.

Dans les exemples 14 à 26 suivants, on a utilisé les catalyseurs préparés de la manière décrite dans les exemples de préparation 1 à 13 pour la synthèse d'un mélange de méthanol et d'alcools supérieurs en $C_2$ à $C_8$ ($C_2$+OH).

EXEMPLE D'UTILISATION 14

On a introduit 1 g du catalyseur préparé dans l'exemple 2 dans un autoclave en acier gainé de verre d'une capacité d'environ 100 ml. On a ensuite introduit un mélange de monoxyde de carbone et d'hydrogène dans un rapport volumétrique de 1 sous une pression initiale à 20°C de 10 MPa dans l'autoclave. On a ensuite chauffé le contenu de l'autoclave pendant 10 heures à 350°C.

Après refroidissement jusqu'à la température ambiante, les produits de réaction ont été condensés et analysés.

On a déterminé les valeurs suivantes :

- Conversion en Co : ce paramètre est exprimé par la relation suivante :

$$\text{Conversion CO} = \frac{\Delta \text{ CO}}{\text{CO entré}} \times 100$$

$$\text{où } \Delta \text{ CO} = CO_{entré} - CO_{sortie} - CO_{2 \text{ formé}}$$

- Sélectivité totale en alcools : ce paramètre $S_A$ exprimé en % est défini par la relation suivante :

$$S_A = \frac{\text{at.g de C en alcool}}{\Delta \text{ CO}}$$

- Sélectivité en alcools lourds : ce paramètre $S_{C_2^+OH}$ exprimé en % est défini par la relation suivante :

$$S_{C_2^+OH} = \frac{C_2^+OH}{CH_3OH + C_2^+OH} \times 100$$

- Sélectivité en produits oxygénés autres que les alcools et l'eau (esters, aldéhydes, cétones, etc.) exprimée par $S_{ox}$ -

$$- \text{ rapport } \frac{C_2^+OH}{CH_3OH}$$

L'analyse des produits obtenus dans plusieurs essais effectués dans les conditions de l'exemple 14 a donné les résultats suivants :

- Conversion CO : 20-25 %
- $S_A = 60\text{-}65$ %
- $S_{C_2^+OH} = 80$ %
- $S_{ox} = 6\text{-}8$ %

$$- \frac{C_2^+OH}{CH_3OH} = 4$$

- Distribution des alcools dans les produits condensés en % en poids :
méthanol : 11-13 %
éthanol : 20-23 %
1-propanol : 11-13 %
1-butanol : 7-9 %
1-pentanol : 4-5 %
1-hexanol : 1,5-2,5 %
1-heptanol : 0,5-1 %
1-octanol : 0,2 %

EXEMPLES D'UTILISATION 15 à 25

On a opéré comme dans l'exemple 14, si ce n'est qu'on a utilisé respectivement dans ces exemples les catalyseurs préparés dans les exemples 1, 3, 4, 5, 6, 7, 8, 10, 11, 12 et 13.

La sélectivité totale ($S_A$) en alcools et la sélectivité ($SC_2^+OH$) en alcools lourds des divers catalyseurs ont été déterminées et sont données dans le tableau II suivant :

TABLEAU II

| Exemples de préparation n° | 1 | 2 | 3 | 4 | 5 | 6 | 7 | 8 | 10 | 11 | 12 | 13 |
|---|---|---|---|---|---|---|---|---|---|---|---|---|
| Exemples d' utilisation n° | 15 | 14 | 16 | 17 | 18 | 19 | 20 | 21 | 22 | 23 | 24 | 25 |
| $S_A$ | 65 | 65 | 70 | 82 | 65 | 75 | 60 | 60 | 58 | 55 | 55 | 75 |
| $S_{C_2}^+OH$ | 75 | 80 | 75 | 50 | 72 | 50 | 81 | 76 | 78 | 80 | 75 | 75 |

EXEMPLE D'UTILISATION 26

On a opéré comme dans l'exemple 14, si ce n'est qu'on a utilisé le catalyseur préparé dans l'exemple 9.

Trois essais indépendants effectués avec ce catalyseur ont donné les sélectivités totales $S_A$ et les sélectivités en alcools lourds $SC_2^+OH$ suivantes :

| Essai n° | 1 | 2 | 3 |
|---|---|---|---|
| $S_A$ | 20 | 60 | 50 |
| $S_{C_2}^+OH$ | 57 | 60 | 71 |

Si l'on compare ces valeurs à celles de l'exemple d'utilisation 21 du tableau II, on constate un manque de reproductibilité dû à l'absence de thorium dans le catalyseur selon l'exemple 9.

De plus, cette comparaison montre que l'absence de thorium diminue la sélectivité en alcool.

Essais comparatifs

On a procédé à des essais visant à utiliser du platine et du palladium dans les catalyseurs suivant l'invention.

Lorsque des dérivés du platine et du palladium sont ajoutés en milieu organique plus particulièrement dans de l'acide formique, ces dérivés sont rapidement réduits sous forme métallique, ce qui empêche une homogénéisation totale du système catalytique.

L'homogénéisation parfaite des catalyseurs polymétalliques suivant l'invention constitue un des principaux avantages du procédé suivant l'invention.

On a procédé également à des essais visant à comparer les performances des catalyseurs suivant la présente invention à ceux décrits dans le document FR-A-2.523.957.

Ces essais ont été effectués dans les conditions décrites dans l'exemple 13, au départ d'un mélange de monoxyde de carbone et d'hydrogène dans un autoclave, en utilisant, d'une part, des catalyseurs à base de cuivre, de cobalt, d'aluminium et d'au moins un métal alcalin ou alcalino-terreux selon le document FR-A-2.523.957 et, d'autre part, des catalyseurs à base de thorium, de cobalt et d'au moins un métal alcalin selon la présente invention.

Ces essais ont révélé qu'en moyenne la sélectivité en alcools lourds $SC_2+OH$ des catalyseurs selon le document FR-A-2.523.957 est de 65 à 70 %, tandis que celle des catalyseurs suivant la présente invention est nettement plus élevée ($SC_2+OH = 75$ à 85 %).

Ces essais ont montré aussi que les sélectivités globales $S_A$ sont très proches l'une de l'autre pour les catalyseurs suivant la présente invention et pour les catalyseurs selon le document FR-A-2.523.957, de même que les conditions d'utilisation de ces deux types de cataly seurs (notamment la pression et la température).

L'augmentation surprenante de la teneur en alcools lourds et donc la diminution de la teneur relative en méthanol peuvent être déterminantes au niveau des applications envisagées puisque, outre qu'ils possèdent un contenu énergétique plus élevé, les mélanges d'alcools obtenus en utilisant les catalyseurs suivant la présente invention ont une meilleure compatibilité avec les hydrocarbures, en sorte qu'il est possible d'augmenter les proportions relatives de ces mélanges d'alcools synthétiques dans les systèmes hydrocarbures-alcools.

Il faut aussi remarquer que la préparation des catalyseurs selon la présente invention est spécialement aisée, particulièrement quand les catalyseurs sont préparés en milieu acide dilué, notamment parce qu'il n'y a pas de contrôle de pH. La stabilité des catalyseurs et la reproductibilité des performances catalytiques sont excellentes.

## Revendications

1. Catalyseurs polymétalliques pour la fabrication d'alcools aliphatiques linéaires en $C_1$ à $C_8$ à partir de gaz de synthèse, caractérisés en ce qu'ils renferment essentiellement du thorium, du cobalt et au moins un métal (A) alcalin éventuellement associé à au moins un métal alcalino-terreux choisis dans le groupe formé par le lithium (Li), le sodium (Na), le potassium (K), le rubidium (Rb), le béryllium (Be), le magnésium (Mg), le calcium (Ca), le strontium (Sr) et le baryum (Ba), les rapports atomiques entre les métaux étant les suivants :
Th/Co = 0,2 à 10
Th/A = 0,01 à 10.

2. Catalyseurs suivant la revendication 1, caractérisés en ce qu'ils contiennent également du zinc, le rapport atomique Th/Zn étant de 0,001 à 1.

3. Catalyseurs suivant la revendication 1, caractérisés en ce que les rapports atomiques entre le thorium, d'une part, et le cobalt et le ou les métaux alcalins ou alcalino-terreux (A) sont les suivants :
Th/Co = 0,4 à 2
Th/A = 0,05 à 1.

4. Catalyseurs suivant la revendication 2, caractérisés en ce que le rapport atomique entre le thorium et le zinc est de 0,01 à 0,5.

5. Catalyseurs suivant l'une quelconque des revendications précédentes, caractérisés en ce que les métaux alcalins et/ou alcalino-terreux (A) sont choisis parmi le sodium, le lithium, le potassium, le magnésium, le calcium et/ou le baryum.

6. Catalyseurs suivant l'une quelconque des revendications précédentes, caractérisés en ce qu'ils contiennent de 1 à 70 % en poids de thorium, de 1 à 60 % en poids de cobalt, de 3 à 50 % en poids de sodium et/ou d'au moins un autre métal alcalin éventuellement associé à au moins un métal alcalino-terreux et de 0 à 90 % en poids de zinc.

7. Catalyseurs suivant la revendication 6, caractérisés en ce qu'ils contiennent de 10 à 70 % en poids de thorium, de 5 à 60 % en poids de cobalt et de 25 à 50 % en poids de sodium et/ou d'au moins un autre métal alcalin éventuellement associé à au moins un métal alcalino-terreux.

8. Catalyseurs suivant la revendication 7, caractérisés en ce qu'ils contiennent environ 40 % en poids de thorium, 15 % en poids de cobalt et 45 % en poids de sodium et/ou d'au moins un autre métal alcalin éventuellement associé à au moins un métal alcalino-terreux.

9. Catalyseurs suivant la revendication 6, caractérisés en ce qu'ils contiennent de 1 à 65 % en poids de thorium, de 1 à 55 % en poids de cobalt, de 2,5 à 50 % en poids de sodium et/ou d'au moins un autre métal alcalin éventuellement associé à au moins un métal alcalino-terreux et de 5 à 90 % en poids de zinc.

10. Catalyseurs suivant la revendication 9, caractérisés en ce qu'ils contiennent environ 12 % en poids de thorium, 5 % en poids de cobalt, 13 % en poids de sodium et/ou d'au moins un autre métal alcalin éventuelle ment associé à au moins un métal alcalino-terreux et 70 % en poids de zinc.

11. Catalyseurs suivant l'une quelconque des revendications précédentes, caractérisés en ce qu'ils sont déposés sous forme de mélanges uniformes de métaux sur au moins un support minéral.

12. Procédé de préparation de catalyseurs suivant l'une quelconque des revendications précédentes, caractérisé en ce qu'on dissout ensemble ou séparément dans au moins un solvant commun ou dans des solvants miscibles l'un à l'autre des composés contenant respectivement du thorium, du cobalt, au moins un métal alcalin éventuellement associé à au moins un métal alcalino-terreux et éventuellement du zinc, en utilisant des proportions telles des divers composés que la solution finale contienne les proportions relatives voulues desdits métaux, on mélange intimement la ou les solutions obtenues, on provoque la précipitation ou le dépôt de dérivés de ces métaux éventuellement sur un support solide insoluble préalablement mélangé à la solution finale, et on active éventuellement le mélange catalytique solide obtenu par traitement thermique.

13. Procédé suivant la revendication 12, caractérisé en ce qu'on opère en milieu acide en mettant les étapes suivantes en oeuvre :
- on dissout un sel solution de thorium dans de l'eau éventuellement additionnée d'un acide carboxylique organique, de manière à obtenir une solution A ;
- on dissout dans un mélange d'eau et d'un acide carboxylique organique, dans lequel au moins un métal alcalin éventuellement associé à au moins un métal alcalino-terreux a été préalablement dissous, un sel soluble de cobalt, de manière à obtenir une solution B ;
- on dissout éventuellement du zinc métallique ou un composé de zinc dans de l'eau et/ou un acide carboxylique organique, de manière à obtenir une solution C ;
- on mélange les solutions A, B et éventuellement C en les agitant de préférence pendant plusieurs heures, après y avoir éventuellement ajouté un support solide insoluble ;
- on évapore la phase liquide sous vide ;
- éventuellement on broie finement le mélange solide obtenu, et
- éventuellement on active la matière solide par chauffage sous vide ou dans un courant de gaz pendant plusieurs heures à une température comprise entre 200 et 1000°C.

14. Procédé suivant la revendication 13, caractérisé en ce qu'on utilise, comme sel soluble de thorium, du formiate de thorium ou du nitrate de thorium hydraté, tandis que comme sel de cobalt, on utilise du carbonate, du nitrate ou de l'acétate de cobalt hydraté.

15. Procédé suivant la revendication 13, caractérisé en ce qu'on utilise un carbonate d'au moins un métal alcalin éventuellement associé à au moins un métal alcalino-terreux.

16. Procédé suivant la revendication 13, caractérisé en ce qu'on utilise du zinc sous forme de zinc métallique en poudre ou sous forme d'oxyde de zinc.

17. Procédé suivant la revendication 13, caractérisé en ce que, comme acide servant de solvant éventuellement en mélange avec de l'eau, on utilise de l'acide formique on de l'acide acétique.

18. Procédé suivant la revendication 12, caractérisé en ce qu'on opère en utilisant un hydrocarbure comme solvant, en mettant les étapes suivantes en oeuvre :
- on dissout un composé de thorium et un composé de cobalt dans un hydrocarbure éventuellement additionné d'un peu d'eau ;
- on chauffe la solution obtenue et, à cette solution agitée, on ajoute soit un support solide et au moins un sel de métal alcalin éventuellement associé à au moins un métal alcalino-terreux, soit un support solide préalablement alcalinisé ;
- on poursuit le chauffage et l'agitation du mélange ;
- on évapore la phase liquide ;
- on soumet éventuellement la matière solide à un fin broyage et
- on l'active éventuellement par chauffage sous vide ou dans un courant de gaz pendant plusieurs heures à une température comprise entre 200 et 1000°C.

19. Procédé suivant la revendication 18, caractérisé en ce que, comme hydrocarbure, on utilise du benzène ou de l'heptane, dans lequel on dissout un composé soluble de thorium choisi, de préférence, parmi l'acétylacétonate de thorium et le formiate de thorium, ainsi qu'un composé soluble de cobalt constitué de préférence par du cobalt carbonyle.

20. Procédé suivant la revendication 18, caractérisé en ce que la solution contenant du thorium et du cobalt est chauffée à une température de 25 à 275°C, de préférence à environ 70°C.

21. Procédé suivant la revendication 12, caractérisé en ce qu'on dissout successivement dans de l'eau un sel de thorium, un sel de cobalt, au moins un sel d'un métal alcalin éventuellement associé à au moins un métal alcalino-terreux et éventuellement un sel de zinc, puis après addition éventuelle d'un support solide insoluble, on agite la solution pendant plusieurs heures et on élimine la phase aqueuse, de préférence par lyophilisation.

22. Procédé de synthèse d'un mélange d'alcools aliphatiques primaires en $C_1$ à $C_8$, caractérisé en ce qu'on fait passer sous pression un gaz de synthèse contenant de l'oxyde de carbone et de l'hydrogène dans des proportions relatives $CO/H_2$ comprises entre 0,2 et 5, de préférence entre 0,5 et 2, sur un catalyseur suivant l'une quelconque des revendications 1 à 11, à une température comprise entre 250 et 420°C.

23. Procédé suivant la revendication 22, caractérisé en ce que la température est comprise entre 300 et 350°C.

**Patentansprüche**

1. Polymetallische Katalysatoren zur Herstellung von linearen aliphatischen $C_1$- bis $C_8$-Alkoholen ausgehend von Synthesegas, dadurch gekennzeichnet, daß sie im wesentlichen Thorium, Kobalt und wenigstens ein Alkalimetall (A), gegebenenfalls in Verbindung mit wenigstens einem Erdalkalimetall, ausgewählt aus der von Lithium (Li), Natrium (Na), Kalium (K), Rubidium (Rb), Beryllium (Be), Magnesium (Mg), Calcium (Ca), Strontium (Sr) und Barium (Ba) gebildeten Gruppe, umfassen, wobei die Atomverhältnisse zwischen den Metallen wie folgt sind:
Th/Co = 0,2 bis 10
Th/A = 0,01 bis 10.

2. Katalysatoren nach Anspruch 1, dadurch gekennzeichnet, daß sie weiterhin Zink enthalten, wobei das Atomverhältnis Th/Zn 0,001 bis 1 ist.

3. Katalysatoren nach Anspruch 1, dadurch gekennzeichnet, daß die Atomverhältnisse zwischen Thorium einerseits und Kobalt und dem oder den Alkali- oder Erdalkalimetallen (A) wie folgt sind:
Th/Co = 0,4 bis 2
Th/A = 0,05 bis 1.

4. Katalysatoren nach Anspruch 2, dadurch gekennzeichnet, daß das Atomverhältnis zwischen Thorium und Zink 0,01 bis 0,5 ist.

5. Katalysatoren nach einem der vorstehenden Ansprüche, dadurch gekennzeichnet, daß die Alkalimetalle und/oder Erdalkalimetalle (A) aus Natrium, Lithium, Kalium, Magnesium, Calcium und/oder Barium ausgewählt sind.

6. Katalysatoren nach einem der vorstehenden Ansprüche, dadurch gekennzeichnet, daß sie 1 bis 70 Gew.-% Thorium, 1 bis 60 Gew.-% Kobalt, 3 bis 50 Gew.-% Natrium und/oder wenigstens eines anderen Alkalimetalls, gegebenenfalls in Verbindung mit wenigstens einem Erdalkalimetall, und 0 bis 90 Gew.-% Zink enthalten.

7. Katalysatoren nach Anspruch 6, dadurch gekennzeichnet, daß sie 10 bis 70 Gew.-% Thorium, 5 bis 60 Gew.-% Kobalt und 25 bis 50 Gew.-% Natrium und/oder wenigstens eines anderen Alkalimetalls, gegebenenfalls in Verbindung mit wenigstens einem Erdalkalimetall, enthalten.

8. Katalysatoren nach Anspruch 7, dadurch gekennzeichnet, daß sie etwa 40 Gew.-% Thorium, 15 Gew.-% Kobalt und 45 Gew.% Natrium und/oder eines anderen Alkalimetalls, gegebenenfalls in Verbindung mit wenigstens einem Erdalkalimetall, enthalten.

9. Katalysatoren nach Anspruch 6, dadurch gekennzeichnet, daß sie 1 bis 65 Gew.-% Thorium, 1 bis 55 Gew.-% Kobalt, 2, 5 bis 50 Gew.-% Natrium und/oder wenigstens eines anderen Alkalimetalls, gegebenenfalls in Verbindung mit wenigstens einem Erdalkalimetall, und 5 bis 90 Gew.-% Zink enthalten.

10. Katalysatoren nach Anspruch 9, dadurch gekennzeichnet, daß sie etwa 12 Gew.-% Thorium, 5 Gew.-% Kobalt, 13 Gew.-% Natrium und/oder wenigstens eines anderen Alkalimetalls, gegebenenfalls in Verbindung mit wenigstens einem Erdalkalimetall, und 70 Gew.-% Zink enthalten.

11. Katalysatoren nach einem der vorstehenden Ansprüche, dadurch gekennzeichnet, daß sie in Form von gleichförmigen Mischungen der Metalle auf wenigstens einen mineralischen Träger aufgebracht sind.

12. Verfahren zur Herstellung der Katalysatoren nach einem der vorstehenden Ansprüche, dadurch gekennzeichnet, daß man in wenigstens einem gemeinsamen Lösungsmittel oder in mischbaren Lösungsmitteln nacheinander jeweils Thorium, Kobalt, wenigstens ein Alkalimetall, gegebenenfalls in Verbindung mit wenigstens einem Erdalkalimetall, und gegebenenfalls Zink enthaltende Verbindungen zusammen oder getrennt löst, wobei man solche Anteile der verschiedenen Verbindungen verwendet, daß die Endlösung die gewünschten relativen Anteile dieser Metalle enthält, die erhaltene(n) Lösung(en) gründlich mischt, die Ausfällung oder Niederschlagung von Derivaten dieser Metalle, gegebenenfalls auf einem unlöslichen festen Träger, der zuvor in die Endlösung eingemischt wurde, herbeiführt und die erhaltene feste katalytische Mischung gegebenenfalls durch thermische Behandlung aktiviert.

13. Verfahren nach Anspruch 12, dadurch gekennzeichnet, daß man im sauren Milieu arbeitet und die folgenden Schritte durchführt:

— Auflösen eines löslichen Thoriumsalzes in gegebenenfalls mit einer organischen Carboxylsäure versetztem Wasser, wobei eine Lösung A erhalten wird;

— Auflösen in einer Mischung aus Wasser und einer organischen Carboxylsäure, in der wenigstens ein Alkalimetall, gegebenenfalls in Verbindung mit wenigstens einem Erdalkalimetall, zuvor gelöst worden ist, eines löslichen Kobaltsalzes, wobei eine Lösung B erhalten wird;

— gegebenenfalls Auflösen von metallischem Zink oder einer Zinkverbindung in Wasser und/oder einer organischen Carboxylsäure, wobei eine Lösung C erhalten wird;

— Mischen der Lösungen A, B und gegebenenfalls C durch Rühren, vorzugsweise über mehrere Stunden, gegebenenfalls nach Zugabe eines unlöslichen festen Trägers;

— Verdampfen der flüssigen Phase im Vakuum;

— gegebenenfalls feines Zerkleinern der erhaltenen festen Mischung und

— gegebenenfalls Aktivieren des festen Materials durch Erhitzen im Vakuum oder in einem Gasstrom über mehrere Stunden auf eine Temperatur zwischen 200 und 1000°C.

14. Verfahren nach Anspruch 13, dadurch gekennzeichnet, daß man als löslichen Thoriumsalz hydratisiertes Thoriumnitrat oder Thoriumformiat verwendet, während man als Kobaltsalz hydratisiertes Kobaltcarbonat, -nitrat oder -acetat verwendet.

15. Verfahren nach Anspruch 13, dadurch gekennzeichnet, daß man das Carbonat wenigstens eines Alkalimetalls, gegebenenfalls in Verbindung mit wenigstens einem Erdalkalimetall, verwendet.

16. Verfahren nach Anspruch 13, dadurch gekennzeichnet, daß man Zink in Form von metallischem Zinkpulver oder in Form von Zinkoxid verwendet.

17. Verfahren nach Anspruch 13, dadurch gekennzeichnet, daß man als Säure, die gegebenenfalls in Mischung mit Wasser als Lösungsmittel dient, Ameisensäure oder Essigsäure verwendet.

18. Verfahren nach Anspruch 12, dadurch gekennzeichnet, daß man unter Verwendung eines Kohlenwasserstoffs als Lösungsmittel arbeitet, wobei man die folgenden Schritte durchführt:

— Auflösen einer Thoriumverbindung und einer Kobaltverbindung in einem Kohlenwasserstoff, der gegebenenfalls mit ein wenig Wasser versetzt ist;

— Erhitzen der erhaltenen Lösung und Zufügen zu der gerührten Lösung entweder eines festen Trägers und wenigstens eines Salzes eines Alkalimetalls, gegebenenfalls in Verbindung mit wenigstens einem Erdalkalimetall, oder eines zuvor mit Alkalien versetzten festen Trägers;

— fortgesetztes Erhitzen und Rühren der Mischung;

— Verdampfen der flüssigen Phase;

— gegebenenfalls feines Zerkleinern des festen Materials und

— gegebenenfalls Aktivieren durch Erhitzen im Vakuum oder in einem Gasstrom über mehrere Stunden bei einer Temperatur zwischen 200 und 1000°C.

19. Verfahren nach Anspruch 18, dadurch gekennzeichnet, daß man als Kohlenwasserstoff Benzol oder Heptan verwendet, wohin man eine lösliche Thoriumverbindung, vorzugsweise ausgewählt aus Thoriumacetylacetonat und Thoriumformiat, und weiterhin eine lösliche Kobaltverbindung, vorzugsweise Kobaltcarbonyl, auflöst.

20. Verfahren nach Anspruch 18, dadurch gekennzeichnet, daß die Thorium und Kobalt enthaltende Lösung auf eine Temperatur von 25 bis 275°C, vorzugsweise auf etwa 70°C, erhitzt wird.

21. Verfahren nach Anspruch 12, dadurch gekennzeichnet, daß man nacheinander im Wasser ein Thoriumsalz, ein Kobaltsalz, wenigstens ein Salz eines Alkalimetalls, gegebenenfalls in Verbindung mit wenigstens einem Erdalkalimetall, und gegebenenfalls ein Zinksalz auflöst, danach, gegebenenfalls nach Zugabe eines unlöslichen festen Trägers, die Lösung über mehrere Stunden rührt und die wäßrige Phase entfernt, vorzugsweise durch Gefriertrocknung.

22. Herstellungsverfahren für eine Mischung von primären aliphatischen $C_1$- bis $C_8$-Alkoholen, dadurch gekennzeichnet, daß man unter Druck ein Kohlenoxid und Wasserstoff in relativen Anteilen von $CO/H_2$ zwischen 0,2 und 5, vorzugsweise zwischen 0,5 und 2, enthaltendes Synthesegas über einen Katalysator nach einem der Ansprüche 1 bis 11 bei einer Temperatur zwischen 250 und 420°C führt.

23. Verfahren nach Anspruch 22, dadurch gekennzeichnet, daß die Temperatur zwischen 300 und 350°C liegt.

## Claims

1. Polymetallic catalysts for the production of $C_1$–$C_8$ linear aliphatic alcohols from synthesis gas, characterized in that they essentially contain thorium, cobalt and at least one alkaline metal (A) possibly associated with at least one alkaline-earth metal selected in the group comprising lithium (Li), sodium (Na), potassium (K), rubidium (Rb), beryllium (Be), magnesium (Mg), calcium (Ca), strontium (Sr) and barium (Ba), the atomic ratios between the metals being the following:

Th/Co = 0.2 to 10

Th/A = 0.01 to 10.

2. Catalysts according to claim 1, characterized in that they also contain zinc, the atomic ratio Th/Zn being 0.001 to 1.

3. Catalysts according to claim 1, characterized in that the atomic ratios between, on one hand, thorium and, on the other hand, cobalt and the alkaline or alkaline-earth metal (A) are the following:

Th/Co = 0.4 to 2
Th/A = 0.05 to 1.

4. Catalysts according to claim 2, characterized in that the atomic ratio between thorium and zinc is 0.01 to 0.5.

5. Catalysts according to anyone of the preceding claims, characterized in that the alkaline and/or alkaline-earth metals (A) are selected among sodium, lithium, potassium, magnesium, calcium and/or barium.

6. Catalysts according to anyone of the preceding claims, characterized in that they contain from 1 to 70% by weight thorium, from 1 to 60% by weight cobalt, from 3 to 50% by weight sodium and/or at least another alkaline metal possibly associated with at least one alkaline-earth metal and from 0 to 90% by weight zinc.

7. Catalysts according to claim 6, characterized in that they contain from 10 to 70% by weight thorium, from 5 to 60% by weight cobalt and from 25 to 50% by weight sodium and/or at least another alkaline metal possibly associated with at least one alkaline-earth metal.

8. Catalysts according to claim 7, characterized in that they contain about 40% by weight thorium, 15% by weight cobalt and 45% by weight sodium and/or at least another alkaline metal possibly associated with at least one alkaline-earth metal.

9. Catalysts according to claim 6, characterized in that they contain from 1 to 65% by weight thorium, from 1 to 55% by weight cobalt, from 2.5 to 50% by weight sodium and/or at least another alkaline metal possibly associated with at least one alkaline-earth metal and from 5 to 90% by weight zinc.

10. Catalysts according to claim 9, characterized in that they contain about 12% by weight thorium, 5% by weight cobalt, 13% by weight sodium and/or at least another alkaline metal possibly associated with at least one alkaline-earth metal and 70% by weight zinc.

11. Catalysts according to anyone of the preceding claims, characterized in that they are deposited in the form of unary mixture of metals on at least one mineral support.

12. Process for preparing catalysts according to anyone of the preceding claims, characterized in that compounds containing respectively thorium, cobalt, at least one alkaline metal possibly associated with at least one alkaline-earth metal and possibly zinc are dissolved together or separately in at least one common solvent or in solvents which are miscible together, by using such proportions of various compounds that the final solution contains the desired selective proportion of metals, the obtained solution(s) is intimately mixed, the precipitation or deposit of derivatives of these metals is made, possibly on an insoluble solid support which has previously been mixed with the final solution, and the obtained solid catalyst mixture is possibly activated by thermal treatment.

13. Process according to claim 12, characterized in that the following steps have been worked in acid medium:

– a soluble salt of thorium is dissolved in water possibly added with an organic carboxylic acid so as to obtain a solution A;

– a soluble salt of cobalt is dissolved in a mixture of water and an organic carboxylic acid in which at least one alkaline metal possibly associated with at least one alkaline-earth metal has previously been dissolved, so as to obtain a solution B;

– metallic zinc or a zinc compound is dissolved in water or in an organic carboxylic acid so as to obtain a solution C;

– solutions A, B and possibly C are mixed by shaking them during several hours, possibly after adding an insoluble solid support;

– the liquid phase is evaporated under vacuum;

– the so-obtained solid mixture is possibly finely ground, and

– the solid material is possibly activated by heating it under vacuum or in a gas flow during several hours at a temperature comprised between 200 and 1000°C.

14. Process according to claim 13, characterized in that hydrated thorium nitrate or thorium formiate is used as soluble salt of thorium, while hydrated cobalt carbonate, nitrate or acetate is used as soluble salt of cobalt.

15. Process according to claim 13, characterized in that a carbonate of at least one alkaline metal possibly associated with at least one alkaline-earth metal is used.

16. Process according to claim 13, characterized in that zinc in the form of metallic zinc powder or zinc oxide is used.

17. Process according to claim 13, characterized in that formic acid or acetic acid is used as acid acting as solvent possibly in mixture with water.

18. Process according to claim 12, characterized in that the following steps have been worked using a hydrocarbon as solvent:

– a thorium compound and a cobalt compound are dissolved in a hydrocarbon possibly added with a little water;

– the obtained solution is heated and to the shaked solution, a solid support and at least one alkaline metal salt possibly associated with at least one alkaline-earth metal or a previously alkalinized solid support is added;

– the heating and the shaking of the mixture are continued;

– the liquid phase is evaporated;

— the solid material is possibly submitted to a finely grinding and is possibly activated by heating it under vacuum or in a gas flow during several hours at a temperature comprised between 200 and 1000°C.

19. Process according to claim 18, characterized in that benzene or heptane is used as hydrocarbon, in which a soluble compound of thorium preferably selected among the thorium acetylacetonate and the thorium formiate, as well as a soluble compound of cobalt preferably constituted of cobalt carbonyl are dissolved.

20. Process according to claim 18, characterized in that the solution containing thorium and cobalt is heated at a temperature from 25 to 275°C, preferably of about 70°C.

21. Process according to claim 12, characterized in that a thorium salt, a cobalt salt, at least an alkaline metal salt possibly associated with at least one alkaline-earth metal and possibly a zinc salt are successively dissolved in water, then possibly after adding an insoluble solid support, the solution is shaked during several hours and the water phase is eliminated, preferably by liophilization.

22. Process for the synthesis of $C_1$ to $C_8$ primary aliphatic alcohols, characterized in that a synthesis gas containing carbone oxide and hydrogen in the relative proportions $CO/H_2$ comprised between 0.2 and 5, preferably between 0.5 and 2, flows on a catalyst according to anyone of claims 1 to 11 at a temperature comprised between 250 and 420°C.

23. Process according to claim 22, characterized in that the temperature is comprised between 300 and 350°C.